# EUROPEAN PATENT SPECIFICATION

(11) **EP 4 351 487 B1**
(45) Date of publication and mention of the grant of the patent: **01.10.2025**
(21) Application number: 22738805.5
(22) Date of filing: 07.06.2022
(51) Int. Cl.: A61F 5/445

(54) **PREBIOTIC, PROBIOTIC, AND/OR POSTBIOTIC TREATMENTS, OSTOMY BARRIERS AND ADHESIVES AND METHODS FOR IMPROVING PERISTOMAL SKIN HEALTH**
PRÄBIOTISCHE, PROBIOTISCHE UND/ODER POSTBIOTISCHE BEHANDLUNGEN, STOMABARRIEREN UND KLEBSTOFFE SOWIE VERFAHREN ZUR VERBESSERUNG DER GESUNDHEIT PERISTOMALER HAUT
TRAITEMENTS PRÉBIOTIQUES, PROBIOTIQUES ET/OU POST-BIOTIQUES, BARRIÈRES DE STOMIE ET ADHÉSIFS, ET PROCÉDÉS POUR AMÉLIORER LA SANTÉ DE LA PEAU PÉRISTOMALE

(30) Priority: 08.06.2021 US 202163208247 P
(43) Date of publication of application: 17.04.2024
(73) Proprietor: Hollister Incorporated, Libertyville, IL 60048 (US)
(72) Inventor: JANIS, Abram D., Libertyville, IL 60048 (US); JOSHI, Jayant, Libertyville, IL 60048 (US); PEDERSEN, Walter R., Libertyville, IL 60048 (US); SHUTT, Joel D., Libertyville, IL 60048 (US); BURT, Rajan A., Libertyville, IL 60048 (US)
(74) Representative: FRKelly
(86) International application number: PCT/US2022/032495
(87) International publication number: WO 2022/261090

(56) References cited:
- WO-A1-2022/031788
- US-A1- 2015 238 543
- US-A1- 2019 175 386

## Description

### BACKGROUND

The present disclosure relates to methods and devices for skin adhesives and ostomy peristomal skin care, more particularly ostomy barriers, adhesives, accessories and treatments that introduce, maintain and/or promote growth of non-pathogenic bacteria or single-celled eukaryotes over pathogenic bacteria or saprophytic fungi.

Skin adhesives are used to secure percutaneous interfaces with the body for a wide variety of medical devices and wearable sensor technologies. An example is ostomy barriers, which are used to secure an ostomy appliance, such as an ostomy pouch, to the skin surrounding the stoma, or "peristomal skin", of the user. Typically, current ostomy barriers include a skin barrier formed from an adhesive, a backing layer and an inlet opening for receiving a stoma. The ostomy barrier may be attached to an ostomy pouch for a one-piece pouch system. Alternatively, the ostomy barrier may be configured as a faceplate for a two-piece pouch system including a body-side coupling that is configured to engage a pouch-side coupling attached to an ostomy pouch. Ostomy accessories are used in conjunction with ostomy barriers and aid in the correct "fit" of the barrier, including adhesion, sealing, removal, and reapplication.

The ostomy barriers may remain attached to a user's skin for extended periods to secure an ostomy pouch. A peristomal skin environment under an ostomy barrier is warm, dark, and moist, and may be intermittently soiled by urine, stool, or other dejecta, which provides an ideal setting for microbial growth that can lead to peristomal skin dysbiosis and/or infections. Studies have found that swabs of the peristomal skin revealed a range of viral, bacterial, and fungal infections as well as colonizing bacterial and fungal microorganisms. Reported negative effects of infection by pathogenic species include impairment of reepithelialization and cellular migration as well as epidermal viability, which may have implications on the healing of peristomal skin complication and conditions. Further, many ostomy patients may be immunosuppressed due to comorbidities, including but not limited to diabetes, adjunctive cancer therapies/chemotherapy, or immunosuppressive/immunomodulatory agents used to treat inflammatory bowel disease, which can further increase the risk of peristomal infections and other complications.

The microbiome, which is the collective genomic population of microorganisms in a particular environment, for example, a person's body or a part of the body, and its effects on health and diseases are an active area of research. Probiotics have been used to selectively promote growth of non-pathogenic microorganisms over pathogenic microorganisms, for example in the gastrointestinal tract and in topical skin care formulations for acne, atopic dermatitis, and other conditions. The non-pathogenic microorganisms are hypothesized to alter the environment and/or offer competition to the pathogenic microorganisms and reduce their relative proportion of the total population. Further, differential culture medium, nutrients and/or conditions have been historically and widely used in the identification and/or isolation of microorganisms and are well characterized in the literature. These same media, nutrients, and/or conditions can be used to selectively promote or suppress specific bacterial, fungal and/or viral species over others.

The present disclosure provides improved skin adhesives, ostomy barriers, appliances, accessories and peristomal treatments comprising non-pathogenic bacteria, fungi, algae and/or ingredients and/or conditions that selectively promote and/or maintain healthy peristomal skin.

US2015238543A1 relates to compositions and methods for targeted killing of microorganisms. In particular, the present disclosure relates to the use of Lysobacter gummosus and compositions containing Lysobacter gummosus in targeted killing of microorganisms in medical, industrial, domestic, or environmental applications, as well as treatment of bacterial infections (e.g., in biofilms).

US2019175386A1 relates to surgical, medical or other anatomical dressings that may be applied to the human body. In particular, the invention relates to a dressing for concealing a structure (in particular, a stoma) on a human body, the dressing comprising a patch arranged to be placed on the body; wherein the patch has an indentation for receiving the structure so as to conceal the structure when the patch is placed on the body.

### BRIEF SUMMARY

The invention is defined in the claims. Any subject-matter that is disclosed herein, but which is not defined in the claims, does not form part of the invention. Claim 1 refers to an ostomy appliance configured to introduce a selected species of non-pathogenic bacteria, fungi, algae or yeast, or modulate existing bacterial populations, or preferentially select or encourage growth of certain non-pathogenic microorganisms over others to promote peristomal skin health according to various embodiments.

The ostomy barrier of the invention comprises a skin barrier or an adhesive and a population or lysate of non-pathogenic bacteria, fungi, algae or yeast that is configured to limit proliferation of pathogenic organisms in or on peristomal skin or maintain healthy skin flora. The population of non-pathogenic bacteria, fungi, algae or yeast, or lysate, may be selected from a group consisting of, for example, but not limited to, *Lactobacillus, Lactobacillus plantarum,* other lactic acid bacteria and *Bifidobacteria,* and lysates thereof.

The ostomy barrier may further include a nutritional medium configured to promote growth of the population of non-pathogenic bacteria, fungi, algae or yeast while suppressing growth of the pathogenic organisms. In an embodiment, the ostomy barrier or adhesive may include a re-sealable opening or a port for introducing the nutritional medium.

In an embodiment, the population or lysate of non-pathogenic bacteria, fungi, algae or yeast may be provided on the skin facing surface of the skin barrier adhesive or incorporated into the barrier or adhesive formulation. For example, the population of non-pathogenic bacteria, fungi, algae or yeast may be provided on a release liner and transferred to the skin facing surface of the skin barrier or adhesive when the release liner is laminated to the skin barrier or adhesive and removed before use.

In an embodiment, the ostomy barrier may be configured to deplete oxygen or provide an air-tight peristomal area to produce an anaerobic environment to limit growth of aerobic bacteria while allowing growth of anaerobic bacteria. Alternatively, an aerobic environment can be provided to limit the growth of anaerobes. In another embodiment, the ostomy barrier or adhesive may be configured to maintain a peristomal skin temperature between about 38°C and 48°C through the use of an integrated heating element. An overlying heating pad may also be utilized to provide selective temperature conditions. In yet another embodiment, the ostomy barrier or adhesive may be configured or formulated to control humidity and/or pH, of the peristomal skin area within selected ranges to promote growth of the population of non-pathogenic bacteria, fungi, algae or yeast while suppressing growth of other or pathogenic organisms. Buffering conditions to maintain specific pH that promotes healthy skin flora over introduced (for example, gut or fecal bacteria) or pathogenic species may also be included.

In some embodiments, the population or lysate of non-pathogenic bacteria, fungi, algae or yeast may be contained in degradable polymeric microspheres or a protein carrier, wherein the degradable polymeric microspheres or the protein carrier may be configured to degrade or breakdown upon exposure to a specific range of ionic condition or a specific range of pH or in the presence of endogenous or pancreatic proteases, as are known to be present in ileostomy and colostomy dejecta. In an embodiment, the population of non-pathogenic bacteria, fungi, algae or yeast may be incorporated and immobilized in a water-soluble carrier formed from polyvinyl alcohol or gelatin or other functionalized polymer providing a preferred environment. In an embodiment, the ostomy accessory or peristomal skin treatment may be provided as a stoma powder or paste.

In yet another aspect, not part of the present invention, a method for improving or maintaining peristomal skin health may comprise the steps of applying a lysate or population of non-pathogenic bacteria, fungi, algae or yeast to peristomal or other skin sites via a vehicle, and attaching an ostomy barrier or adhesive, respectively, configured to promote growth of the population of non-pathogenic bacteria, fungi, algae or yeast.

In an embodiment, the lysate and/or population of non-pathogenic bacteria, fungi, algae or yeast may be provided in a liquid form, which may be sprayed or wiped on the peristomal skin or other skin sites, or provided in a gel, paste or powder form. The adhesive or ostomy barrier may comprise a nutritional medium configured to promote growth of the population of non-pathogenic bacteria, fungi, algae or yeast while suppressing growth of pathogenic organisms. In an embodiment, the ostomy barrier may be configured to alter at least one aspect of the peristomal skin, such as, nutrients, oxygenation, ionic concentration, temperature, hydration, humidity, or pH, in order to promote growth of the population of non-pathogenic bacteria, fungi, algae or yeast while suppressing growth of selected pathogenic organisms.

In an embodiment, an ostomy barrier may incorporate solid, gel, and/or liquid additives to regulate microclimate humidity. For example, desiccants or vapor pressure regulating additives may be incorporated into an ostomy barrier, allowing for the regulation of humidity within the peristomal skin microenvironment. The barrier may be tailored to specific humidity conditions through the selection of differing additive agents and/or desiccants and their proportion within the barrier. Additional humidity specification may be expanded by combination mixtures of additive agents, differing in chemical identity and proportion within the barrier. In another embodiment, a humidity regulating adhesive, skin protectant, or device may be applied as an accessory for peristomal care. Humidity regulation may be customized to promote growth of beneficial microbial populations, to suppress pathogenic organisms, or in a combination thereof.

### DETAILED DESCRIPTION

The microbiome of human skin differs across anatomical sites due to microclimates, for example, dry, sebaceous, and moist microclimates. A surgically created ostomy disrupts the gut and/or skin microbiome to various degrees, potentially altering the innate microbiomes therein. It also creates a unique microclimate at an interface between skin and gut for a potentially unique and dysbiotic mixed skin/gut microbiome. The present disclosure discloses methods and devices to preserve, modulate, or alter an ostomy environment to maintain and/or reestablish peristomal skin health according to various embodiments.

Probiotics as used herein are living microorganisms which are administered to confer health benefits on a host. The health benefits of probiotics may depend on a bacterial strain, delivery or application method, and clinical conditions.

Prebiotics as used herein are compounds or ingredients that may selectively stimulate growth and/or activity of one or more strains of microorganisms that are beneficial to the health of a host.

Postbiotics as used herein refer to soluble factors (products or metabolic byproducts) secreted by live microorganisms or released after microbial lysis, such as enzymes, peptides, teichoic acids, peptidoglycan-derived muropeptides, polysaccharides, cell surface proteins, and organic acids.

Monocultures are described in the present disclosure for simplification, but co- or multi-culture of more than one bacterial species, fungal, algal species or a combination thereof may also be used.

In an embodiment, an ostomy appliance or accessory (for example, an ostomy barrier, stoma ring, stoma powder, etc.) may be configured to deliver a single bacterial species or a mixture of bacteria or a single or mixture of lysates at one time or in a sequence to maintain and improve the health of peristomal skin. The bacteria may be delivered in various forms, for example, planktonic (e.g. in a liquid suspension which may be sprayed, wiped or poured onto the skin), freeze dried powder, and/or immobilized spores on a solid substrate, such as an ostomy barrier comprising hydrocolloids or other biomaterials, which may be applied to the peristomal skin and isolate the skin from ostomy effluent. Suitable genera of microbes for the peristomal skin applications may include, but are not limited to, *Lactobacillus, Lactobacillus plantarum,* lactic acid bacteria and *Bifidobacteria,* or lysates thereof. Such microbes are currently used as over-the-counter (OTC) probiotics, and thus, are readily available. Many other bacterial, fungal and eukaryotic species including algae may also be suitable for the peristomal skin applications, for example *Staphylococcus* and/or *Streptococcus.*

A bacterial culture or inoculum of non-pathogenic bacteria, or its lysates thereof may be applied to at-risk, irritated, breached, colonized or infected peristomal skin in order to competitively colonize the region and prevent adhesion, colonization, and proliferation of pathogenic bacteria. Examples of application methods may include sprays, swabs, dressing-immobilized cultures or spore strips. In some embodiments, selective nutrition or conditions for a selected non-pathogenic organism(s) may be incorporated into a delivery means to aid in proliferation of the selected bacteria, algae, yeast, fungi, or the like. In an embodiment, biofilm forming cocktails of organisms or specific species that limit growth of other species through a release of natural antibiotics or competing for nutrients or attachment sites may be introduced to the peristomal skin via an ostomy barrier, accessory or other application methods according to various embodiments.

In an embodiment, an ostomy skin barrier may contain a lysate or population of bacteria, algae, yeast or fungi to be applied to the peristomal skin in the form of an additive, gel or component. In another embodiment, bacteria or bacterial lysates may be formulated into a spray dried powder format which may be used directly as a stoma powder, or integrated into a wipe formulation, an adhesive composition, an adhesive tape composition, a seal or paste formulation, and the like. Reservoirs, gels or degradable microspheres may be utilized as a delivery vehicle to alter peristomal conditions over time.

In some embodiments, a bacterial formulation may be integrated into an ostomy pouch film to reduce odor causing bacteria and/or to promote a skin friendly environment. For example, an ostomy pouch may contain a population of non-pathogenic bacteria, fungi, algae, yeast, or their lysates. In another example, an ostomy pouch may be formed from a polymeric film comprising a population of non-pathogenic bacteria, fungi, algae, yeast, or their lysates Such ostomy pouches may facilitate a healthy microbiome environment even when a user experiences a backflow of pouch contents. In an embodiment, an ostomy pouch may be formed from a multilayer film including an inner layer comprising a population of non-pathogenic bacteria, fungi, algae, yeast, or their lysates.

In an embodiment, a lysate or population of selected bacteria, algae, yeast or fungi may first be applied to the peristomal skin, for example, via spray or powder application, followed by an ostomy barrier configured to maintain desirable conditions for the applied population of bacteria, algae, yeast or fungi.

In an embodiment, an ostomy appliance or accessory may be configured to selectively encourage growth of a preferred species or a mixture of preferred species, or selectively discourage growth of an undesirable species or a mixture of undesirable species. For example, the peristomal skin treatment or the ostomy appliance or accessory may include prebiotics, postbiotics, and/or selective modulation of interfacial/environmental conditions to control microbiota in the peristomal skin and acute or chronic dermal wounds and skin conditions, such as atopic dermatitis or psoriasis. The ostomy appliance or accessory may or may not contain or deliver live bacteria or microbes.

In some embodiments, an ostomy barrier may comprise a specific growth medium or may otherwise alter a peristomal skin environment to preserve or encourage growth of one species of bacteria while discouraging growth of others. As used herein, a peristomal skin environment may include, but is not limited to, ionic concentration, temperature, pH, humidity and hydration level of the peristomal skin. For example, a hydrocolloid ostomy barrier may comprise materials that promote growth of a particular species or a class of organisms while limiting growth of others through providing a selective nutritional composition, inducing hyperoxia/hypoxia/anoxia, or changing the pH of the peristomal skin. Such an ostomy barrier may be applied to an at-risk, infected, or inoculated peristomal skin site to establish, limit or preserve a differential organism population. In an embodiment, an ostomy appliance may include a skin barrier material comprising a nutritional medium configured to selectively promote growth of a population of non-pathogenic bacteria, fungi, algae, yeast, or their lysates, while suppressing growth of pathogenic organisms.

In an embodiment, an ostomy barrier or accessory may be configured to modulate a relative population of aerobic bacteria and anaerobic bacteria to improve peristomal skin health. For example, an ostomy barrier may be configured to deplete oxygen or provide an air-tight peristomal area to produce an anaerobic environment that can limit growth of aerobic bacteria but allow or encourage growth of anaerobes.

In an embodiment, an ostomy barrier may be configured to control the peristomal skin temperature within a range, using an integrated heating element powered by a battery, a capacitor, or other power source, wherein the temperature range is selected for a differential survival of bacteria of the genus *Lactobacillus* (e.g., 43-46°C), which is outside the temperature range for successful growth of pathogenic *Staphylococcus aureus* (optimum temperature 37°C) and *Staphylococcus epidermidis* (optimum temperature between 26°C and 37°C), or *Pseudomonas aeruginosa* (optimum temperature 37°C).

In an embodiment, an ostomy barrier may comprise a re-sealable opening or port to introduce a nutritional medium configured to promote the growth of a selected bacteria as needed. In such an embodiment, inoculation of the bacteria may also be performed without changing the ostomy barrier.

In an embodiment, an ostomy barrier may include an inlet port, an outlet port, humidity regulating reservoirs, or a combination thereof, which may be configured to control humidity or concentrations of gases. In such an embodiment, the ostomy barrier may include a gauge for measuring or detecting a level of humidity or gases. Regulation of the environmental humidity may allow for dynamic modification of the peristomal microbiome environment. For example, lowering a relative humidity of the peristomal area may upregulate driving pressures for a dry skin microbiome, as these driving pressures may be more comparable to the microenvironment of healthy, non-sebaceous skin tissue. In an embodiment, a peristomal treatment may desiccate the peristomal skin to differentially select dehydration tolerant species over pathogenic species that grow in a moist environment, such as waterborne bacterium (e.g. *Pseudomonas aeruginosa).*

In an embodiment, an ostomy barrier may comprise materials that contain or release specific carbon sources to preserve or encourage growth of one or more bacteria species while discouraging growth of others. In some embodiments, reservoirs, gels, or degradable microspheres may be used to deliver or alter the peristomal skin conditions over time. For example, degradable polymeric microspheres containing bacteria, spores, medium, media components, may be triggered to release based on degradation of the carrier which may be tuned based on ionic conditions of the skin (hypo/iso/hypertonic) or a pH level. In another example, a protein carrier may contain bacteria, spores, medium, and/or media components, wherein the protein carrier may be configured to breakdown in the presence of endogenous proteases, the concentration of which may be elevated in irritated, inflamed or infected peristomal skin or exogenous proteases, such as those found in ileostomy and to a lesser extent, colostomy dejecta. The protein carrier may also be configured to breakdown in the presence of enzymes introduced from the stoma, for example pancreatic proteases. Alternatively, degradable carbohydrate-based or lipid-based polymers may be used to deliver bacteria, spores, medium, or media components in the presence of endogenous or pancreatic amylases or lipases, respectively.

Probiotic organism or prebiotic compounds may also be incorporated and immobilized in a water-soluble carrier, such as polyvinyl alcohol (PVOH) or a gelatin or other functionalized polymeric microcapsule. In an embodiment, the immobilized probiotic organism or prebiotic compound may be applied to a release liner and laminated to an ostomy barrier, wherein the probiotic organism or prebiotic compound may be transferred to a skin facing surface of the ostomy barrier when the release liner is removed before use. When the ostomy barrier is attached to the peristomal skin of a user, the immobilized probiotic organism or prebiotic compound may be placed at an interface between the ostomy barrier and the user's skin, wherein the probiotic organism or prebiotic compound may be released by exposure to moisture, perspiration or stoma effluent. In some embodiments, a mixture of prebiotic organisms and probiotic compounds may be provided on a skin facing surface of an ostomy barrier to promote growth of beneficial microorganisms.

The pH level of the peristomal skin may be controlled to within a range that is selective for a differential survival of bacteria of the genus *Lactobacillus* (e.g., below pH 5.0, which is outside a pH range for successful growth of pathogenic *Staphylococcus aureus* (pH optimum 7.4-7.6), *Staphylococcus epidermidis* (pH optimum 6.8), or *Pseudomonas aeruginosa* (pH optimum 6.6-7.0)). In some embodiments, both pH level and temperature of the peristomal skin may be controlled to within selected ranges. In an embodiment, a skin adhesive, peristomal treatment, ostomy barrier or accessory may comprise a material that metabolizes to produce metabolite that lowers pH for a selective survival of *Lactobacillus* species

In an embodiment, an ostomy barrier or accessory may be configured to provide a hypertonicity (high salt) which may be differentially selective for Lactobacillus over other bacterial species, such as Pseudomonas and Staphylococcus. A hypertonic condition may be created, for example, through a brining process with NaCl, for a selective survival of Lactobacillus species over other bacterial flora.

In some embodiments, yeast or algae may be introduced to the peristomal skin in addition to or alternative to bacteria of the genus Lactobacillus. In such embodiments, a peristomal treatment or an ostomy barrier may be configured to provide a peristomal environment that encourages growth of the yeast or algae. Yeast species suitable for peristomal skin applications may include Saccharomyces cerivisae (used in brewing of beer), which has a pH optimum of 4 and a temperature optimum of 26°C. Suitable algae species may include those commonly used in food production such as Clorella (pH optimum 10-10.5, temperature optimum 15°C, low salinity), Spirulina (temperature optimum 32°C, pH 9-9.5), and marine species that have evolved in seawater which may have optimal growth in hypertonic (0.6M) conditions.

According to various embodiments of the present disclosure, competitive organisms may be introduced to occupy binding sites within the exposed extracellular matrix (ECM) of the peristomal skin and compete for nutrients and other resources to limit proliferation of pathogenic species. In some embodiments, extremophilic Archaebacteria may also be utilized, as these bacteria thrive in non- physiologic conditions that may not be detrimental to healing or maintenance of healthy peristomal skin. These species can persist in high salt and high or low pH environments, as well as in temperature extremes that may be selective against typical pathogenic wound bacteria or fungi.

In the present disclosure, the words "a" or "an" are to be taken to include both the singular and the plural. Conversely, any reference to plural items shall, where appropriate, include the singular.

From the foregoing it will be observed that numerous modifications and variations can be effectuated without departing from the scope of the novel concepts of the present disclosure. It is to be understood that no limitation with respect to the specific embodiments illustrated is intended or should be inferred. The disclosure is intended to cover by the appended claims all such modifications as fall within the scope of the claims

## Claims

1. An ostomy appliance comprising a skin barrier material and **characterised in** comprising a population of non-pathogenic bacteria, fungi, algae, yeast, or their lysates ("the population"), wherein the population is configured to limit proliferation of pathogenic organisms and/or selectively enhance the proliferation and maintenance of non-pathogenic or commensal bacteria in a peristomal skin environment.

2. The ostomy appliance of claim 1, wherein the population is provided on a skin facing surface of the skin barrier material or incorporated into the skin barrier material.

3. The ostomy appliance of claims 1 or 2, further comprising a nutritional medium configured to selectively promote growth of the population, while suppressing growth of the pathogenic organisms.

4. The ostomy appliance of any of claims 1-3, wherein the population is provided on a release liner, wherein the release liner is laminated to the skin barrier material, wherein the population is transferred to a skin facing surface of the skin barrier material.

5. The ostomy appliance of any of claims 1-3, wherein the skin barrier material is formed from a skin barrier formulation incorporating the population, wherein the population migrates to a skin facing surface of the skin barrier material.

6. The ostomy appliance of any of claims 1-5, wherein the population is selected from a group consisting of Lactobacillus, Lactobacillus plantarum, lactic acid bacteria and Bifidobacteria, and lysates thereof.

7. The ostomy appliance of any of claims 1-5, wherein the population is selected from a group consisting of Saccharomyces cerivisae, Clorella, Spirulina, Archaebacteria, and lysates thereof

8. An ostomy appliance of any of claims 1-7, wherein the ostomy appliance is configured to deplete oxygen or provide an air-tight peristomal area to produce an anaerobic environment to limit growth of aerobic bacteria while allowing growth of anaerobic bacteria.

9. An ostomy appliance of any of claims 1-7, wherein the ostomy appliance is configured to provide oxygen to produce an aerobic environment to limit growth of anaerobic bacteria while allowing growth of aerobic bacteria.

10. The ostomy appliance of any of claims 1-9, wherein ostomy appliance is configured to maintain a peristomal skin temperature between about 38°C and 48°C.

11. The ostomy appliance of any of claims 1-10, further comprising a re- sealable opening or a port for introducing a nutritional medium configured to promote growth of the population while suppressing growth of other or pathogenic organisms.

12. The ostomy appliance of any of claims 1-11, wherein the ostomy appliance is configured to control a humidity of the peristomal skin area within a selected range to promote growth of the population while suppressing growth of the pathogenic organisms.

13. The ostomy appliance of any of claims 1-12, wherein the ostomy appliance is configured to control a pH level of the peristomal skin area within a selected range to promote growth of the population while suppressing growth of the pathogenic organisms.

14. The ostomy appliance of any of claims 1-13, wherein the ostomy appliance is an ostomy barrier or an ostomy ring.

15. The ostomy appliance of any of claims 1-13, wherein the ostomy appliance is an ostomy pouch system, wherein the ostomy pouch system includes an ostomy pouch formed from a polymeric film comprising the population.

## Patentansprüche

1. Stomavorrichtung, umfassend ein Hautbarrierematerial und **dadurch gekennzeichnet, dass** sie eine Population nicht pathogener Bakterien, Pilze, Algen, Hefen oder deren Lysate ("die Population") umfasst, wobei die Population zum Begrenzen einer Vermehrung pathogener Organismen und/oder zum selektiven Fördern der Vermehrung und Aufrechterhaltung nicht pathogener oder kommensaler Bakterien in einer peristomalen Hautumgebung ausgelegt ist.

2. Stomavorrichtung nach Anspruch 1, wobei die Population auf einer der Haut zugewandten Fläche des Hautbarrierematerials bereitgestellt oder in das Hautbarrierematerial eingearbeitet ist.

3. Stomavorrichtung nach Anspruch 1 oder 2, ferner umfassend ein Nährmedium, das zum selektiven Fördern von Wachstum der Population bei gleichzeitigem Unterdrücken von Wachstum der pathogenen Organismen ausgelegt ist.

4. Stomavorrichtung nach einem der Ansprüche 1-3, wobei die Population auf einer Abziehfolie bereitgestellt ist, wobei die Abziehfolie auf das Hautbarrierematerial laminiert ist, wobei die Population auf eine der Haut zugewandte Fläche des Hautbarrierematerials übertragen wird.

5. Stomavorrichtung nach einem der Ansprüche 1-3, wobei das Hautbarrierematerial aus einer Hautbarriereformulierung gebildet ist, in die die Population eingearbeitet ist, wobei die Population zu einer der Haut zugewandten Fläche des Hautbarrierematerials wandert.

6. Stomavorrichtung nach einem der Ansprüche 1-5, wobei die Population ausgewählt ist aus einer Gruppe, bestehend aus Lactobacillus, Lactobacillus plantarum, Milchsäurebakterien und Bifidobakterien sowie deren Lysaten.

7. Stomavorrichtung nach einem der Ansprüche 1-5, wobei die Population ausgewählt ist aus einer Gruppe, bestehend aus Saccharomyces cerivisae, Clorella, Spirulina, Archaebakterien sowie deren Lysaten.

8. Stomavorrichtung nach einem der Ansprüche 1-7, wobei die Stomavorrichtung zum Abreichern von Sauerstoff oder zum Bereitstellen eines luftdichten peristomalen Bereichs zum Erzeugen einer anaeroben Umgebung ausgelegt ist, um Wachstum von aeroben Bakterien bei gleichzeitigem Ermöglichen von Wachstum von anaeroben Bakterien zu begrenzen.

9. Stomavorrichtung nach einem der Ansprüche 1-7, wobei die Stomavorrichtung zum Bereitstellen von Sauerstoff zum Erzeugen einer aeroben Umgebung ausgelegt ist, um Wachstum von anaeroben Bakterien bei gleichzeitigem Ermöglichen von Wachstum von aeroben Bakterien zu begrenzen.

10. Stomavorrichtung nach einem der Ansprüche 1-9, wobei die Stomavorrichtung zum Aufrechterhalten einer peristomalen Hauttemperatur zwischen etwa 38 °C und 48 °C ausgelegt ist.

11. Stomavorrichtung nach Anspruch 1-10, ferner umfassend eine wiederverschließbare Öffnung oder einen Port zum Einbringen eines Nährmediums, das zum Fördern von Wachstum der Population bei gleichzeitigem Unterdrücken von Wachstum anderer oder pathogener Organismen ausgelegt ist.

12. Stomavorrichtung nach einem der Ansprüche 1-11, wobei die Stomavorrichtung zum Steuern von Feuchtigkeit des peristomalen Hautbereichs innerhalb eines ausgewählten Bereichs zum Fördern von Wachstum der Population bei gleichzeitigem Unterdrücken von Wachstum der pathogenen Organismen ausgelegt ist.

13. Stomavorrichtung nach einem der Ansprüche 1-12, wobei die Stomavorrichtung zum Steuern eines pH-Niveaus des peristomalen Hautbereichs innerhalb eines ausgewählten Bereichs zum Fördern von Wachstum der Population bei gleichzeitigem Unterdrücken von Wachstum der pathogenen Organismen ausgelegt ist.

14. Stomavorrichtung nach einem der Ansprüche 1-13, wobei die Stomavorrichtung eine Stomabarriere oder ein Stomaring ist.

15. Stomavorrichtung nach einem der Ansprüche 1-13, wobei die Stomavorrichtung ein Stomabeutelsystem ist, wobei das Stomabeutelsystem einen Stomabeutel beinhaltet, der aus einer Polymerfolie gebildet ist, die die Population umfasst.

## Revendications

1. Appareil de stomie comprenant un matériau de barrière de la peau et **caractérisé en ce qu'**il comprend une population de bactéries, de champignons, d'algues, de levures non pathogènes, ou de leurs lysats (« la population »), dans lequel la population est configurée pour limiter la prolifération d'organismes pathogènes et/ou améliorer sélectivement la prolifération et le maintien de bactéries non pathogènes ou commensales dans un environnement de la peau péristomale.

2. Appareil de stomie selon la revendication 1, dans lequel la population est disposée sur une surface faisant face à la peau du matériau de barrière de la peau ou incorporée dans le matériau de barrière de la peau.

3. Appareil de stomie selon les revendications 1 ou 2, comprenant également un support nutritionnel configuré pour favoriser sélectivement la croissance de la population, tout en supprimant la croissance des organismes pathogènes.

4. Appareil de stomie selon l'une quelconque des revendications 1 à 3, dans lequel la population est disposée sur une doublure antiadhésive, dans lequel la doublure antiadhésive est laminée sur le matériau de barrière de la peau, dans lequel la population est transférée sur une surface faisant face à la peau du matériau de barrière de la peau.

5. Appareil de stomie selon l'une quelconque des revendications 1 à 3, dans lequel le matériau de barrière de la peau est formé à partir d'une formulation de barrière de la peau incorporant la population, dans lequel la population migre vers une surface faisant face à la peau du matériau de barrière de la peau.

6. Appareil de stomie selon l'une quelconque des revendications 1 à 5, dans lequel la population est choisie à partir d'un groupe constitué de Lactobacillus, Lactobacillus plantarum, de bactéries lactiques et de Bifidobactéries, et de leurs lysats.

7. Appareil de stomie selon l'une quelconque des revendications 1 à 5, dans lequel la population est choisie à partir d'un groupe constitué de Saccharomyces cerivisae, Clorella, Spirulina, Archaebactéries, et de leurs lysats.

8. Appareil de stomie selon l'une quelconque des revendications 1 à 7, dans lequel l'appareil de stomie est configuré pour épuiser l'oxygène ou fournir une zone péristomale étanche à l'air pour produire un environnement anaérobie pour limiter la croissance de bactéries aérobies tout en permettant la croissance de bactéries anaérobies.

9. Appareil de stomie selon l'une quelconque des revendications 1 à 7, dans lequel l'appareil de stomie est configuré pour fournir de l'oxygène pour produire un environnement aérobie pour limiter la croissance de bactéries anaérobies tout en permettant la croissance de bactéries aérobies.

10. Appareil de stomie selon l'une quelconque des revendications 1 à 9, dans lequel l'appareil de stomie est configuré pour maintenir une température de la peau péristomale entre environ 38 °C et 48°C.

11. Appareil de stomie selon l'une quelconque des revendications 1 à 10, comprenant également une ouverture refermable ou un port pour l'introduction d'un support nutritionnel configuré pour favoriser la croissance de la population tout en supprimant la croissance d'autres organismes pathogènes.

12. Appareil de stomie selon l'une quelconque des revendications 1 à 11, dans lequel l'appareil de stomie est configuré pour commander une humidité de la zone de la peau péristomale dans une plage sélectionnée pour favoriser la croissance de la population tout en supprimant la croissance des organismes pathogènes.

13. Appareil de stomie selon l'une quelconque des revendications 1 à 12, dans lequel l'appareil de stomie est configuré pour commander un niveau de pH de la zone de la peau péristomale dans une plage choisie pour favoriser la croissance de la population tout en supprimant la croissance des organismes pathogènes.

14. Appareil de stomie selon l'une quelconque des revendications 1 à 13, dans lequel l'appareil de stomie est une barrière de stomie ou un anneau de stomie.

15. Appareil de stomie selon l'une quelconque des revendications 1 à 13, dans lequel l'appareil de stomie est un système de poche de stomie, dans lequel le système de poche de stomie comporte une poche de stomie formée à partir d'un film polymère comprenant la population.
